# FASCICULE DE BREVET EUROPEEN

(11) **EP 3 089 597 B1**
(45) Date de publication et mention de la délivrance du brevet: **22.08.2018**
(21) Numéro de dépôt: 14820898.6
(22) Date de dépôt: 29.12.2014
(51) Int. Cl.: A23L 33/10

(54) **COMPOSITION DIETETIQUE POUR DIMINUER L'INFLAMMATION DE BAS GRADE COMPRENANT DE LA DHA.**
DHA ENTHALTENDE DIÄTETISCHE ZUSAMMENSETZUNG ZUR VERMINDERUNG VON LOW-GRADE-ENTZÜNDUNGEN
DHA CONTAINING DIETETIC COMPOSITION FOR REDUCING LOW-GRADE INFLAMMATION

(30) Priorité: 30.12.2013 FR 1363718
(43) Date de publication de la demande: 09.11.2016
(73) Titulaire: PRI, S. A., 4963 Clémency (LU)
(72) Inventeur: ZAHOUANI, Abdelhadi, F-95100 Argenteuil (FR)
(74) Mandataire: Vidon Brevets & Stratégie
(86) Numéro de dépôt international: PCT/EP2014/079391
(87) Numéro de publication internationale: WO 2015/101608

(56) Documents cités:
- WO-A1-2007/043870
- WO-A1-2008/045739
- WO-A2-2007/002837
- WO-A2-2007/073176
- WO-A2-2008/012329
- WO-A2-2009/153064
- WO-A2-2010/136900
- CN-A- 102 429 307
- DATABASE CAPLUS [Online] CHEMICAL ABSTRACTS SERVICE, COLUMBUS, OHIO, US; 19 mars 2013 (2013-03-19), Siriwardhana et al.: "Modulation of adipose tissue inflammation by bioactive food compounds", XP002726421, extrait de STN Database accession no. 2013:411713
- DATABASE CAPLUS [Online] CHEMICAL ABSTRACTS SERVICE, COLUMBUS, OHIO, US; 12 juillet 2011 (2011-07-12), Makhoul et al.: "Association of obesity with triglycerides and C-reactive protein are attenated in adults with high red blood cell eicosapentaenoic and docoshexaenoic acids", XP002726422, extrait de STN Database accession no. 2011:867032
- DATABASE CAPLUS [Online] CHEMICAL ABSTRACTS SERVICE, COLUMBUS, OHIO, US; 19 juillet 2010 (2010-07-19), Moreno-Aliaga et al.: "Regulation of adipokine secretion by n-3 fatty acids", XP002726423, Database accession no. 2010:894011
- KELLY ET AL.: "Long-chain polyunsaturated fatty acids may mutually benefit both obsity and osteoporosis", NUTRITION RESEARCH, vol. 33, 12 avril 2013 (2013-04-12), pages 521-533, XP002726424,
- CALDER P C ET AL: "Polyunsaturated fatty acids, inflammation and immunity", EUROPEAN JOURNAL OF CLINICAL NUTRITION, NATURE PUBLISHING GROUP, GB, vol. 56, no. SUPPL. 3, 1 août 2002 (2002-08-01), pages S14-S19, XP002466790, ISSN: 0954-3007, DOI: 10.1038/SJ.EJCN.1601478
- DATABASE CAPLUS [Online] CHEMICAL ABSTRACTS SERVICE, COLUMBUS, OHIO, US; 4 mai 2012 (2012-05-04), RUTAO et al.: "Method for manufacturing low-calorie complete nutrient health drink", XP002726425, extrait de STN Database accession no. 2012:640438
- SIRIWARDHANA NALIN ET AL: "Modulation of adipose tissue inflammation by bioactive food compounds", THE JOURNAL OF NUTRITIONAL BIOCHEMISTRY, ELSEVIER, AMSTERDAM, NL, vol. 24, no. 4, 15 March 2013 (2013-03-15) , pages 613-623, XP028996584, ISSN: 0955-2863, DOI: 10.1016/J.JNUTBIO.2012.12.013
- Z MAKHOUL ET AL: "Associations of obesity with triglycerides and C-reactive protein are attenuated in adults with high red blood cell eicosapentaenoic and docosahexaenoic acids", EUROPEAN JOURNAL OF CLINICAL NUTRITION, vol. 65, no. 7, 1 July 2011 (2011-07-01), pages 808-817, XP055394671, ISSN: 0954-3007, DOI: 10.1038/ejcn.2011.39
- MARÍA J. MORENO-ALIAGA ET AL: "Regulation of adipokine secretion by n-3 fatty acids", PROCEEDINGS OF THE NUTRITION SOCIETY, vol. 69, no. 03, 14 June 2010 (2010-06-14) , pages 324-332, XP055394675, GB ISSN: 0029-6651, DOI: 10.1017/S0029665110001801

## Description

### 1. Domaine de l'invention

Le domaine de l'invention est celui des compositions alimentaires de régime. Plus précisément, l'invention concerne une composition alimentaire de régime, destinée à être consommée dans le cadre d'un régime de type très basses calories permettant de diminuer l'inflammation de bas grade sur le court terme (entre 1 et 3 mois) et le long terme (sur plus d'un an).

### 2. Art antérieur

Selon les chiffres de l'OMS, l'obésité a doublé depuis 1980 et le surpoids concerne environ 1,4 milliards de personnes de 20 ans et plus (estimations de 2008) (http://www.who.int/mediacentre/factsheets/fs311/fr/index.html). D'après des estimations de 2011, environ 40 millions des enfants de moins de 5 ans seraient en surpoids. Or, l'OMS recense environ 3 millions de décès directement liés à la condition de surpoids et d'obésité, ce qui en font le cinquième facteur de risque de décès dans le monde. Actuellement, il semblerait que la tendance de ces chiffres soit à la croissance.

Les notions d'obésité et de surpoids sont communément appréciées par l'Indice de Masse Corporel (IMC) qui est le rapport entre le poids en kilogramme et le carré de la taille en mètre : IMC = masse (kg)/ taille² (m). Un patient est considéré en surpoids lorsque son IMC est supérieur ou égal à 25. L'obésité est déclarée lorsque l'IMC est supérieur ou égal à 30 et l'obésité morbide est déterminée pour un IMC supérieur ou égal à 35 ou 40.

Obésité et surpoids ont diverses étiologies : mutations génétiques, dérèglement hormonal... mais elles sont le plus souvent liées à une mauvaise hygiène de vie, à savoir un apport calorique nettement plus important que la dépense énergétique du sujet.

L'obésité et le surpoids sont également sources de différents problèmes : troubles articulaires, essoufflement, difficulté à se mouvoir, fatigue excessive, dépression nerveuse due à une mauvaise image de soi, sentiment de rejet par la société etc. Outre ces problèmes, ce sont également des facteurs de risque non négligeables pour la santé du patient et notamment pour les maladies cardio-vasculaires, l'hypertension, certaines formes de cancer et le diabète de type II. De plus, la prise en charge de ces patients et de leurs pathologies représente environ 2 à 7% des dépenses des systèmes de santé pour les pays développés. Par conséquent, obésité et surpoids sont devenus des problèmes de santé publique à l'échelle mondiale.

Sur le plan moléculaire, on a récemment découvert que les obèses présentent une augmentation modérée mais chronique des taux circulants de médiateurs de l'inflammation comme l'interleukine 6 (IL6), le fibrinogène, la protéine réactive C (PRC), le facteur de nécrose tumorale plus couramment désigné sous sa traduction anglaise « tumor necrosis factor alpha » (TNFα), le sérum amyloïde de type A (SAA) etc. Ce phénomène est symptomatique d'une inflammation chronique et modérée ou inflammation de bas grade.

De nombreuses études ont permis de corréler l'augmentation du taux de TNFα et IL6 à l'augmentation de la masse grasse, autorisant désormais la communauté scientifique à considérer l'obésité comme un état inflammatoire chronique tout comme les pathologies fréquemment associées à l'obésité : diabète de type II et athérosclérose par exemple.

On constate également que certains régimes amaigrissants proposés permettent de diminuer légèrement la concentration en PRC, TNFα et IL6 chez le patient obèse. Toutefois, ces constatations sont à prendre avec précaution : les effets d'un régime à très faibles calories n'ont été étudiées que sur de courtes périodes d'environ 3 semaines, chez des patients obèses présentant un diabète de type II. De plus, des résultats d'étude ont démontré que, malgré une perte de poids considérable et une diminution du tour de taille importante indiquant une perte de masse grasse, les niveaux d'IL6 et TNFα et autres cytokines pro-inflammatoires ne reviennent pas à un niveau normal. Par conséquent, l'inflammation chronique persiste malgré la perte de poids et la perte de masse grasse. Or, la prise de poids et l'inflammation chronique sont intimement liées. Cette persistance de l'inflammation de bas grade est à l'origine du phénomène de rebond, ou reprise de poids, observé chez les patients obèses ou en surpoids à la fin d'un régime très basses calories.

Or il est important de limiter cette reprise de poids, fréquemment désignée par l'expression « effet yoyo » ou « effet rebond ». L'effet rebond est à l'origine de l'obésité et d'épisodes dépressifs aggravés par le sentiment de frustration et d'isolement social suite aux échecs des régimes. L'effet rebond est en grande partie lié à la persistance de l'état d'inflammation chronique qui n'est pas résolu par le simple régime. De plus, l'effet rebond décourage le patient qui ressent comme une frustration et un échec cette reprise de poids malgré ses efforts importants. En outre, le patient tente alors de perdre à nouveau le poids repris en refaisant un régime. Or, l'organisme finit par résister aux régimes consécutifs. De plus, certains patients se contraignent à adopter une alimentation hypocalorique sur le long terme et s'infligent par conséquent de nombreuses privations alimentaires dans l'angoisse de reprendre. Ces situations aggravent l'isolement social et la condition psychologique fragile des patients obèses.

Le document WO2009/153064 (Nutricia NV) divulgue des compositions alimentaires liquides prêtes à consommer et enrichies en EPA/DHA, pour utiliser par dees patients obèses, notamment dans le cadre d'une chrurgie bariatrice de ces patients. Les compositions de ce document s'utilisent entre autres pour traiter la micro-inflammation chroniques des patients obèses.

### 3. Objectifs de l'invention

L'invention a notamment pour objectif de pallier ces inconvénients de l'art antérieur.

Plus précisément, un objectif de l'invention est de fournir, dans au moins un mode de réalisation, une composition alimentaire de régime permettant de réduire l'inflammation de bas grade, c'est à dire une inflammation chronique et modérée, chez le patient en surpoids ou obèse.

Un autre objectif de l'invention est de fournir, dans au moins un mode de réalisation, des portions alimentaires individuelles à consommer par le sujet dans le cadre d'un régime de type à très faibles calories.

L'invention a également pour objectif de fournir, dans au moins un mode de réalisation, une composition alimentaire ou des portions alimentaires individuelles permettant de limiter le risque de reprise de poids.

### 4. Exposé de l'invention

Ces objectifs, ainsi que d'autres qui apparaîtront par la suite, sont atteints à l'aide d'une composition alimentaire de régime prête à l'emploi pour diminuer l'inflammation de bas grade chez le patient obèse ou en surpoids comprenant, par rapport au poids total de la composition:
- entre 70% et 80% en poids de protéines ;
- entre 4% et 6% en poids de glucides ; et,
- entre 2% et 5% en poids de lipides;
lesdits lipides incluant entre 0,25% et 2% d'acide docosahexaénoïque (DHA) en poids par rapport audit poids de lipides.

Ainsi, l'invention repose sur une approche tout à fait nouvelle et originale de proposer une composition alimentaire comprenant un pourcentage élevé d'un acide gras polyinsaturé particulier de la famille dite des omégas 3 : l'acide docosahexaénoïque (DHA). On sait que les omégas 6 ont un rôle pro-inflammatoire tandis que les omégas 3 auraient tendance à diminuer le niveau des marqueurs de l'inflammation dans le sang. Les spécialistes recommandent généralement de conserver ce rapport oméga 6/ oméga 3 inférieur à 5. Or, le rapport observé dans l'alimentation des sujets en surpoids ou obèses est généralement supérieur à 10. Les quantités d'oméga 3 préconisées pour rétablir le rapport entre oméga 6/ oméga 3 à une valeur inférieur à 5, lutter contre la prise de poids et protéger le système cardiovasculaire sont impossibles à obtenir uniquement par l'alimentation. Les inventeurs ont donc conçu une composition alimentaire de régime permettant de couvrir cet apport en oméga 3. Ainsi, la composition alimentaire selon l'invention permet de diminuer sur le court et le long terme, à savoir à partir du premier mois de consommation et sur plusieurs années, la concentration des marqueurs de l'inflammation et de diminuer, voire éliminer, l'inflammation de bas grade chez les patients obèses ou en surpoids. Grâce à la résolution de l'état inflammatoire, la perte de poids est facilitée tout en évitant le phénomène de rebond à la fin du régime. Le régime amaigrissant est donc plus efficace.

L'originalité de la présente invention réside donc en ce que les compositions et les portions alimentaires permettent non seulement la perte de poids, mais également d'éviter toute reprise ou effet rebond grâce à la résolution de l'inflammation de bas grade. Il est important de distinguer les différents « types » d'inflammation. On entend par « inflammation chronique de bas grade » une inflammation diffuse dans l'organisme entier du sujet, sans foyer inflammatoire précis et sans gonflement, chaleur, rougeur et douleur. Aucun foyer inflammatoire n'est apparent. Les cellules du système immunitaire et les substances inflammatoires sont présentes de manière diffuse dans l'organisme et endommagent les cellules saines du sujet. Ces substances inflammatoires peuvent être dosées dans le sang et les cellules du système immunitaires observées au microscope. Cette inflammation peut être causée par une infection de départ persistante, suite à une infection virale par exemple. Elle peut également être causée par un excès de sucre et/ou de graisse dans l'alimentation. L'obésité est considérée depuis peu comme une inflammation de bas grade systémique, tout comme de nombreuses pathologies qui lui sont associées telles que l'athérosclérose, le diabète de type II etc.

Jusqu'à présent, aucune publication n'a fait état de compositions alimentaires ou de portions alimentaires permettant d'éviter le phénomène de rebond ou effet yoyo par la diminution de l'inflammation systémique de bas grade. La composition selon l'invention comprend, par rapport au poids total de l'invention :
- entre 70% et 80% en poids de protéines ;
- entre 4% et 6 % en poids de glucides ; et,
entre 2% et 5 % en poids de lipides; lesdits lipides incluant entre 0,25% et 2% d'acide docosahexaénoïque (DHA) en poids par rapport audit poids de lipides.

Avantageusement, la composition selon l'invention est utilisée pendant une période comprise entre 30 jours et 180 jours par le patient, de préférence entre 60 jours et 180 jours, de manière davantage préférée entre 90 jours et 180 jours. Cette durée peut être déterminée par le médecin en fonction des résultats de plusieurs paramètres du patient comme la mesure de la perte de poids, la mesure du tour de taille, les dosages lipidiques ou les dosages des marqueurs de l'inflammation. Toutefois, il est préférable de consommer ces portions pendant au moins 30 jours. En effet, bien qu'une perte de poids peut être observée au bout de 2 ou 3 semaines, il est préférable de consommer les portions selon l'invention pendant au moins un mois afin de constater les premiers effets de la DHA sur la résolution de l'inflammation chronique de bas grade, même si une perte de poids est observée dès les premiers jours de régimes. Les études scientifiques ont démontré que le mécanisme de la résolution de l'inflammation liée à la sécrétion des médiateurs lipidiques de l'inflammation dérivés des acides gras nécessite plusieurs jours pour être déclenché (C.N. Serhan, Novel Lipid Mediators and Resolution Mechanisms in Acute Inflammation : To Resolve or Not ?, The American Journal of Pathology, vol.177(4), 4 October 2010, pp :1576-1591) Plus exactement, plus la consommation des portions selon l'invention est prolongée, moins les marqueurs pro-inflammatoires sont sécrétés et plus les marqueurs anti-inflammatoires sont exprimés.

Un objet de l'invention concerne en outre une portion alimentaire individuelle de régime pour diminuer l'inflammation de bas grade comprenant :
- 12,5 g à 20 g, de préférence 14 g à 16 g, de protéines et,
- 0,5 g à 5 g, de préférence 1 g à 5 g, de glucides ; et,
- 0,25 à 6 g, de préférence entre 0,5 et 4 g, de lipides ; lesdits lipides incluant jusqu'à 80 mg de DHA.

Ces quantités sont variables dans le cadre de l'invention tant que leurs pourcentages tombent dans les gammes revendiquées.

Les portions individuelles peuvent donc constituer un en-cas, un repas ou une partie du repas. Il est prévu, dans le cadre d'un régime de type à très faibles calories suivi par un médecin, de faire varier la quantité quotidienne de DHA prise par le patient. On entend par régime à très basses calories un régime dans lequel l'apport calorique quotidien du patient est limité à 800 kcals par jour au maximum.

Dans les premières semaines de son régime, le patient commencera à prendre entre 500-600 mg de DHA par jour.

Les portions alimentaires selon l'invention peuvent se présenter sous la forme d'une poudre à mélanger avec de l'eau par exemple pour obtenir un bouillon, une soupe, une boisson chaude ou froide, tels un chocolat, un café, un jus de fruits, une omelette...

Les portions alimentaires selon l'invention peuvent également se présenter sous forme d'aliments prêt à consommer comme, et de manière non exhaustive, des barres de céréales ou barres aromatisées, des galettes, des biscuits, des crêpes, des sauces, des céréales pour petit-déjeuner. Il est important pour le sujet de conserver des aliments dont la forme est identique ou proche d'une alimentation conventionnelle afin d'éviter qu'il se lasse et abandonne son régime. Cela permet également de ne pas le couper de son environnement social en l'empêchant de partager son repas avec des amis ou sa famille. Les portions individuelles facilitent aussi le dosage, c'est à dire que la quantité de nourriture est plus simple à calculer pour le patient puisqu'il lui suffit de compter le nombre de portions à manger pendant un repas. En effet, certains individus prennent du poids ou n'arrivent pas à mincir en mangeant équilibré parce qu'ils ont tendance à consommer des quantités de plus en plus importantes de nourriture sans se rendre compte qu'ils dépassent leurs besoins énergétiques quotidiens. Enfin, les portions individuelles sont faciles à transporter : le sujet peut donc prendre avec lui une barre comme en-cas dans la journée ce qui lui permettra de combler une faim sans commettre d'écarts ou de préparer son déjeuner sur son lieu de travail.

Un autre avantage des portions alimentaires selon l'invention est qu'elles facilitent le calcul de la dose quotidienne de protéines et de DHA à consommer par le patient. En effet, la quantité de DHA qu'un praticien ou un diététicien prescrit peut varier en fonction des besoins du patient, de sa résistance relative à consommer des compositions ou des portions alimentaires de régime, de la perte de poids envisagée etc. En ce cas, il est intéressant pour un patient de pouvoir calculer rapidement sa dose quotidienne de DHA en se basant tout simplement sur le nombre de portions consommées dans la journée. Il est ainsi libre de les répartir à sa guise en fonction de son mode de vie. Il est donc plus simple d'observer pour le patient la dose quotidienne de DHA prescrite par le médecin en se basant sur le nombre de portions journalières conseillées. Ainsi, le patient peut suivre son régime sans frustration ni contrainte.

Il est à noter que ce dernier avantage est également obtenue avec une composition alimentaire selon l'invention. Par exemple, la composition alimentaire selon l'invention peut se présenter sous forme d'une poudre en vrac accompagnée d'une cuillère de dosage. Le patient n'aura alors qu'à compter le nombre de cuillérées de composition consommées dans la journée.

Avantageusement, la portion alimentaire individuelle de régime selon l'invention comprend entre 1 mg et 80 mg de DHA, de préférence entre 10 et 50 mg de DHA, de manière davantage préférée entre 25 mg et 50 mg de DHA et de manière préférée entre toutes 50 mg de DHA.

Selon l'invention, la portion alimentaire individuelle de régime présente un poids compris entre 5g et 300g, alternativement entre 15g et 300g, de préférence entre 15g et 200g, de manière davantage préférée entre 20g et 125g.

Les portions selon l'invention peuvent prendre la forme de lots comprenant par exemple plusieurs éléments de 5g ou 10g, par exemple un petit sachet de biscuits.

De préférence, la portion alimentaire individuelle a été réalisée à partir d'ingrédients culinaires dont au moins un contient du DHA. Il est préférable de formuler les portions alimentaires individuelles à partir de sources alimentaires naturelles plutôt que d'inclure une forme synthétique de DHA. En effet, l'incorporation sous forme d'ingrédients culinaires permet de formuler plus facilement les portions selon l'invention. De plus, il est important que les portions alimentaires soient savoureuses et agréables à déguster afin de faciliter l'observance du régime.

Avantageusement, la portion selon l'invention est utilisée pendant une période comprise entre 30 jours et 180 jours par le patient, de préférence entre 60 jours et 180 jours, de manière davantage préférée entre 90 jours et 180 jours. La durée de consommation des portions alimentaires individuelles est fonction du poids de départ et du nombre de kilos à perdre. Toutefois, il est préférable de consommer ces portions pendant au moins 30 jours. En effet, bien qu'une perte de poids peut être observée au bout de 2 ou 3 semaines, il est préférable de consommer les portions selon l'invention pendant au moins un mois afin de constater les premiers effets de la DHA sur la résolution de l'inflammation chronique de bas grade. Plus exactement, plus la consommation des portions selon l'invention est prolongée, moins les marqueurs de l'inflammation sont sécrétés et plus les marqueurs anti-inflammatoires sont exprimés.

Dans un mode de réalisation avantageux, ledit ingrédient culinaire contenant du DHA est choisi parmi le jaune d'oeuf, l'huile de poisson, les extraits d'algues, les champignons, le krill ou la combinaison d'au moins deux de ces ingrédients. Ces ingrédients sont d'importantes sources naturelles de DHA.

Dans un mode de réalisation intéressant, lesdites protéines sont des protéines choisies parmi les protéines de lait, de lactosérum, de soja, d'oeuf ou leur combinaison.

Selon l'invention, la composition alimentaire de régime prête à l'emploi peut être obtenue par le mélange d'une portion telle que décrite précédemment avec de l'eau. Ainsi, la composition alimentaire de régime est un repas obtenu en mélangeant une portion alimentaire, telle une soupe ou une boisson lyophilisée, à de l'eau. Parfois, certains mélanges peuvent être chauffés.

On qualifie de « prêt à l'emploi » une composition alimentaire ne nécessitant pas de cuisson ou un temps de cuisson au four à micro-ondes qui n'est pas supérieur à 5 minutes. Une composition alimentaire de régime prête à l'emploi pourrait également être obtenue par l'association d'une portion individuelle alimentaire avec au moins une gélule de DHA, ladite gélule comprenant entre 100 mg et 300mg de DHA. Ainsi, le patient pourrait également choisir de conserver une alimentation plus traditionnelle et d'atteindre la dose quotidienne de DHA qui lui est recommandée en associant une portion individuelle avec des gélules de DHA, pour former une composition alimentaire de régime prête à l'emploi. L'invention a également pour objet l'utilisation de DHA pour la préparation d'une composition alimentaire de régime pour diminuer l'inflammation de bas grade chez le patient obèse ou en surpoids.

Avantageusement, l'utilisation de DHA permet de préparer une composition alimentaire de régime comprenant, par rapport au poids total de la composition,
- entre 70% et 80% en poids de protéines ;
- entre 4% et 6 % en poids de glucides ; et,
- entre 2% et 5 % en poids de lipides;
lesdits lipides incluant entre 0,25% et 2% de DHA par rapport au poids total des

### 5. Liste des figures

D'autres caractéristiques et avantages de l'invention apparaîtront plus clairement à la lecture de la description suivante d'un mode de réalisation préférentiel, donné à titre de simple exemple illustratif et non limitatif, et des dessins annexés, parmi lesquels :
- la figure 1 est un graphique présentant l'évolution de la perte de poids chez des patients ayant suivi un régime à très basses calories, avec ou sans supplémentation de DHA, à J30, J60 et J180 ;
- la figure 2 est un graphique représentant l'évolution du tour de taille chez les sujets des deux groupes étudiés, à J30, J60 et J180 ;
- la figure 3 est un graphique illustrant l'évolution du marqueur pro-inflammatoire PRCus entre les deux groupes ;
- la figure 4 est un graphique illustrant l'évolution du taux de leptine entre les deux groupes;
- la figure 5 est un graphique illustrant l'évolution du taux de PD1 entre les deux groupes;
- la figure 6 est un graphique présentant les variations du taux de 14HDOHE entre les deux groupes étudiés ;
- la figure 7 est un graphique illustrant la variation, pour chacun des groupes de patients, de la concentration en molécules pro-inflammatoires et anti-inflammatoires entre J0 et J180 ;
- la figure 8 est un graphique représentant l'évolution du ratio ARA/DHA pour chaque groupe de patient à J30, J60 et J180 ; et
- la figure 9 est un graphique illustrant l'évolution du taux de l'index d'acides gras anti-inflammatoires (AIFAI) pour chaque groupe de patient à J30, J60 et J180.

### 6. Description d'un mode de réalisation de l'invention

Le principe général de l'invention repose sur l'incorporation d'un oméga 3 particulier, la DHA, dans des compositions alimentaires de régime pour réduire, voire éliminer, l'inflammation de bas grade chez les sujets obèses ou en surpoids. Selon l'invention, la composition alimentaire peut se présenter sous la forme d'une portion alimentaire individuelle, sous forme de poudre en vrac à mélanger avec de l'eau ou sous forme d'un aliment prêt à consommer telle une barre, un biscuit, une brique de boisson etc.

On décrit ci-dessous des exemples de portions alimentaires similaires aux compositions de l'invention telle que revendiquée, à l'exception de leur teneur en glucides qui est supérieure à 6% dans toutes les compositions exemplifiées ci-dessous (6.1 - 6.5).

### 6.1 Exemple de portion individuelle: Moka

La portion selon l'invention peut se présenter sous la forme d'une poudre destinée à être mélangée avec de l'eau froide pour obtenir un café Moka. La portion présente un poids total de 25g et comprend :
- protéines : 15g
- lipides : 0,6g dont 50mg de DHA
- glucides : 3,8g
- fibres : 1,7g
- minéraux : 1,9g.

La boisson peut également comprendre entre 1 et 2 g de sels minéraux pour le bon équilibre du sujet tels que du calcium, du phosphore, du sodium, du potassium, du magnésium, du fer, du zinc, du cuivre, de l'iode, du manganèse, du sélénium, du chrome, du molybdène et du fluor. La boisson peut également être enrichie en vitamines et en acides aminés essentiels.

### 6.2 Exemple de portion individuelle: gâteau à la pomme

Le gâteau présente un poids d'environ 27g et comprend :
- protéines : 15 g
- lipides : 1,6g dont 50mg de DHA
- glucides : 2,9 g
- fibres : 5,1 g
- minéraux: 1 g

Le gâteau comprend en outre des sels minéraux et tous les acides aminés essentiels.

### 6.3 Exemple de portion individuelle: cacao pour petit-déjeuner

La portion selon l'invention peut se présenter soit sous la forme d'une poudre destinée à être mélangée avec de l'eau froide pour obtenir une boisson au cacao ; soit se présenter sous la forme d'une boisson individuelle prête à consommer et à emporter. La portion présente un poids total de 26g et comprend :
- protéines : 15 g
- lipides : 2,2 g dont 50 mg de DHA
- glucides : 1,7 g
- fibres : 4,2 g
- minéraux : 1,7 g.

La boisson au cacao selon l'invention comprend en outre des sels minéraux dont du calcium (214,4 mg), du phosphore (171,6 mg), du sodium (249,4 mg), du potassium (179,2 mg), du magnésium (32,5 mg) et du fer (1,1 mg).

### 6.4 Exemple de portion individuelle: nuggets de poulet

Chaque nugget présente un poids d'environ 27g et comprend :
- protéines : 15 g
- lipides : 1,9 g dont 50mg de DHA
- glucides : 2,5 g
- fibres : 4,9 g
- minéraux : 1 g

Chaque nugget comprend environ 545mg de sels minéraux dont du calcium, du phosphore, du sodium, du potassium, du magnésium, du fer et du zinc.

### 6.5 Exemple de portion individuelle: omelette saveur fromage

L'omelette selon l'invention se présente sous la forme d'un sachet de poudre de 27g, qu'il suffit de mélanger avec de l'eau avant cuisson. La poudre comprend :
- protéines : 15 g
- lipides : 2,9 g dont 50mg de DHA
- glucides : 3,4 g
- fibres : 2,1 g
- minéraux : 1,9 g

La poudre pour omelette comprend en outre environ 830 mg de sels minéraux (calcium, phosphore, sodium, potassium, magnésium, fer et zinc).

### 6.6 Résultats comparatifs entre des sujets ayant suivi une cure d'amaigrissement comprenant ou non de la DHA.

Une étude sous contrôle médical a été menée chez des patients obèses (IMC moyen = 33). Les sujets ont été séparés en deux groupes :
- un groupe témoin consommant les compositions alimentaires similaires à celles de l'invention, mais dépourvues de DHA; et
- un groupe « étude » consommant les compositions alimentaires selon l'invention, comprenant de la DHA.

A leur entrée dans le programme ainsi qu'à 30 jours éventuellement, à 60 jours et au bout de 6 mois, les sujets ont subi une prise de sang afin d'évaluer leur taux de marqueurs pro- ou anti-inflammatoires en fonction de leur prise ou perte de poids. Les marqueurs ou molécules indiquant la présence d'une inflammation recherchés sont les leucocytes, les neutrophiles, la protéine C réactive (PRC), le TNF-alpha, l'interleukine 6, la leptine, la resistine et l'adiponectine. Les marqueurs lipidiques indiquant une régression de l'inflammation ou ayant une activité anti-inflammatoires recherchés sont 6kPGF1-alpha (6-céto-prostaglandine F1-alpha), TxB2 (tromboxane B2), PGE2 (prostaglandine E2), LxB4 (lipoxine B4), LxA4 (lipoxine A4), RvD1 (résolvine D1), LtB5 (leukotriène B5), 7MaResin (Maresine 7), Pdx (Protectine dx), LtB4 (leukotriène B4), 18-HEPE (acide 18-hydroxyeicosapentaenoique), 15-HETE (acide 15-hydroxyeicosatetraenoique), 17-HDoHE (acide 17-docosahexaenoique), 14-HDoHE (acide 14-docosahexaenoique), 8-HETE (acide 8-hydroxyeicosatetraenoique), 12-HETE (acide 12-hydroxyeicosatetraenoique) et 5-HETE (acide 5-hydroxyeicosatetraenoique). Ce sont des médiateurs lipidiques dérivés de la DHA.

Pendant les 60 premiers jours de l'étude, les patients du groupe «étude » ont chacun reçu 500 mg de DHA par jour, par l'unique biais de la consommation des compositions et portions alimentaires selon l'invention. Leur apport calorique a été inférieur à 800 kcals par jour et s'est principalement présenté sous la forme de compositions ou portions alimentaires selon l'invention. Plus exactement, les patients ont consommé 5 portions alimentaires selon l'invention dans la journée dont 2 lors des principaux repas accompagnés de végétaux, et ce afin d'assurer leur apport de DHA notamment. Après 60 jours, leur apport calorique a été progressivement augmenté par l'introduction de différents groupes d'aliments jusqu'à atteindre un niveau calorique compris entre 1300 et 2000 kcals/jour. L'objectif est de rééduquer le patient sur le plan alimentaire. Les aliments traditionnels ont été progressivement réintroduits dans l'alimentation quotidienne du patient, afin d'éviter de carencer et lasser le patient d'une part et de rééduquer le patient sur le plan alimentaire.

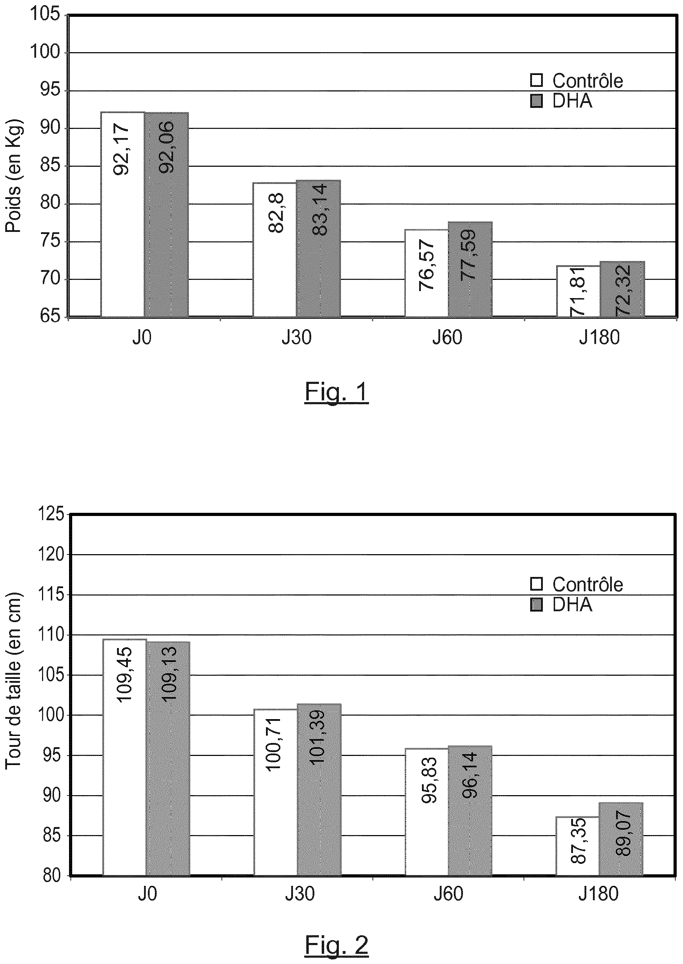

Comme on peut le voir sur la figure 1, les sujets des deux groupes ont perdu entre 15 et 16 kg en moyenne en l'espace de 60 jours en suivant le programme d'amaigrissement, et environ 20 kg en 6 mois (J180) depuis le début de la cure à J0. L'évolution du tour de taille chez les deux groupes de patients est représentée par le graphique de la figure 2. Le tour de taille permet d'évaluer la perte de graisse abdominale, paramètre associé au syndrome métabolique. Comme on peut l'observer, les sujets de chacun des groupes ont perdu environ 15 cm de tour de taille en moyenne au bout de 60 jours et entre 20 et 22 cm après 6 mois de cure d'amaigrissement. La similarité des résultats sur la perte de poids et l'évolution du tour de taille s'explique par le fait que les patients ont suivi le même régime hypocalorique, indépendamment de l'apport de DHA.

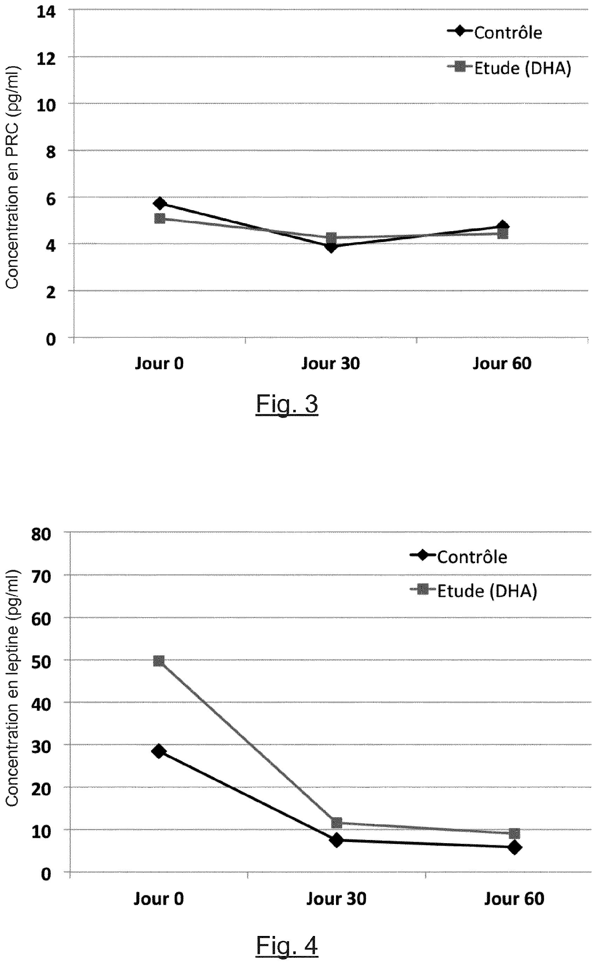

Au vu des figures 3 et 4, on constate une diminution des marqueurs pro-inflammatoires. Deux marqueurs de l'inflammation associée à l'obésité ont été recherchés et dosés : la protéine C réactive ultrasensible (PRCus), et la leptine.

La perte de poids permet de diminuer de manière globale le taux de PRC chez les sujets des 2 groupes (figure 3). Plus exactement, une diminution de 12% a été mesurée pour les patients du groupe contrôle tandis qu'une diminution de 18% environ a été obtenue pour les patients ayant reçu de la DHA quotidiennement pendant leur cure d'amaigrissement.

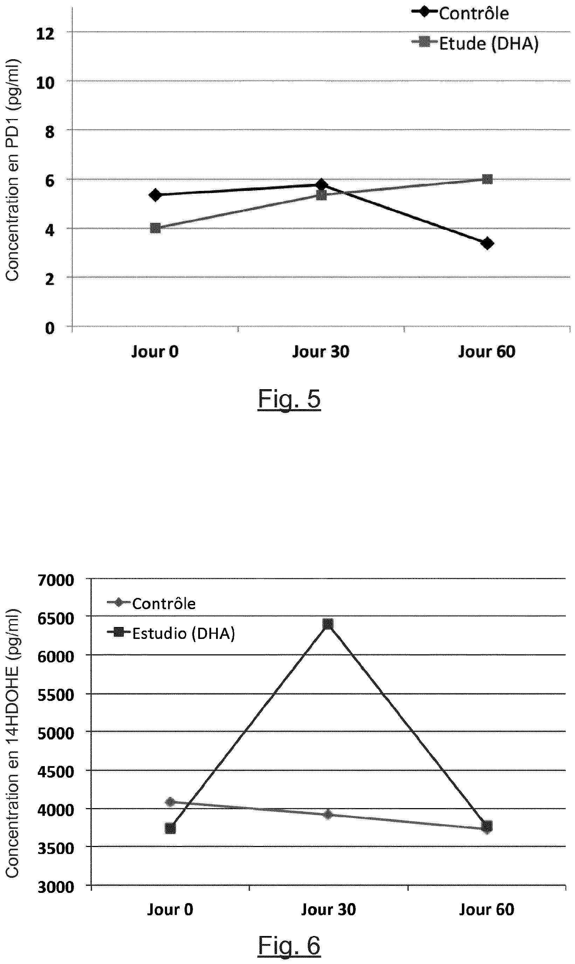

Au vu de la figure 4, on constate que le groupe « étude » présente un taux de leptine deux fois supérieur à celui du groupe « contrôle ». La leptine participe, entre autres, à la régulation de l'appétit et à l'homéostasie de la réponse immunitaire. La leptine est surexprimée chez les patients en surpoids ou obèses. Comme on peut l'observer, le taux de leptine des sujets du groupe « contrôle » est divisé par 3 grâce à leur perte de poids et de masse grasse. Les patients du groupe « étude » ont en revanche obtenu des résultats plus satisfaisants car leur taux de leptine a été divisé en moyenne par un facteur 5, grâce à l'absorption quotidienne de DHA (p<0,001 à 60 jours). Les figures 5 et 6 présentent les résultats obtenus sur l'amélioration de l'expression des marqueurs anti-inflammatoires PD1 et 14-HDOHE. Dans les deux groupes, on constate une augmentation du taux de ces marqueurs dans le sang. Comme on peut l'observer sur le graphique de la figure 5, le taux de PD1 n'est pas amélioré chez les patients du groupe contrôle, malgré leur perte de poids : il est même diminué de 36% depuis le début de leur cure. Ce résultat n'implique pas que l'inflammation progresse chez le patient mais qu'elle perdure et s'installe dans la chronicité sans évoluer vers sa résolution. Au contraire, les patients ayant reçu de la DHA pendant leur cure voient leur taux de PD1 augmenter régulièrement (+ 83% de PD1 au jour 60 par rapport au jour 0). Ce résultat démontre que les compositions de régime comprenant de la DHA permettent non seulement de freiner l'inflammation liée à l'obésité mais également de faire évoluer l'inflammation vers sa résolution. On peut également constater au vu de la figure 6 que les sujets consommant de la DHA régulièrement ont un taux de 14HDOHE significativement plus élevé que les sujets du groupe témoin (p < 0,00095) au bout de 30 jours.

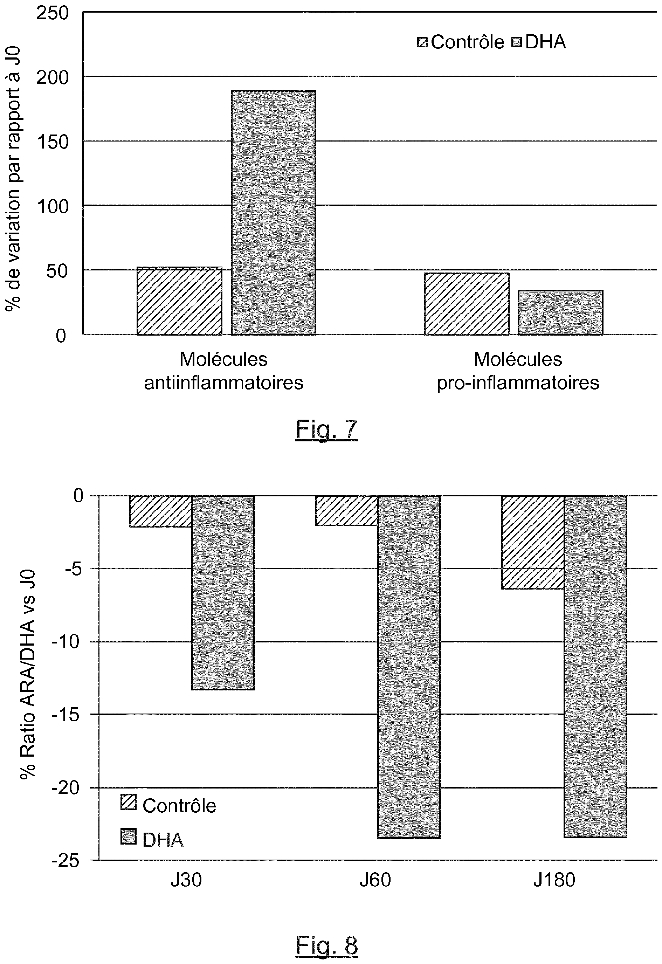

La figure 7 présente l'évolution globale et moyenne des marqueurs pro- et anti-inflammatoires pour chaque groupe de patients après 6 mois de cure d'amaigrissement. Brièvement, chaque patient a subi une prise de sang pour évaluer la concentration en différents marqueurs avant d'entamer sa cure d'amaigrissement. Au bout de 6 mois, les patients ont à nouveau subi une prise de sang pour mesurer ces mêmes marqueurs. Comme on peut l'observer sur la figure 7, les patients ayant reçu une supplémentation quotidienne en DHA présentent un taux environ 4 fois supérieur en molécules anti-inflammatoires par rapport au groupe contrôle. De même, la prise de DHA permet également de réduire la quantité de marqueurs de l'inflammation par rapport au groupe contrôle.

La figure 8 présente l'évolution du ratio Acide Arachidonique (noté ARA)/ DHA au cours du temps, par rapport au ratio initial mesuré chez les patients avant d'entamer leur cure. L'acide arachidonique (ARA) est un métabolite clé de l'inflammation. Cet acide gras non essentiel sert de précurseurs à de nombreux marqueurs de l'inflammation. Comme on peut l'observer au vu de la figure 8, dès les 30 premiers jours d'amaigrissement, le ratio ARA/DHA devient négatif pour les deux groupes de patients. Toutefois, cette diminution est beaucoup plus importante et marquée chez les patients consommant de la DHA. En effet, au vu des résultats présentés, même après 180 jours de régime, le ratio ARA/ DHA des patients du groupe contrôle présente un niveau de diminution d'environ 50% par rapport à celui des patients avec DHA à J30. La présence de DHA dans le groupe étude permet de freiner la lipolyse et par la suite inhiber la sécrétion accrue des acides gras dont l'acide arachidonique, ce qui permet de diminuer l'inflammation.

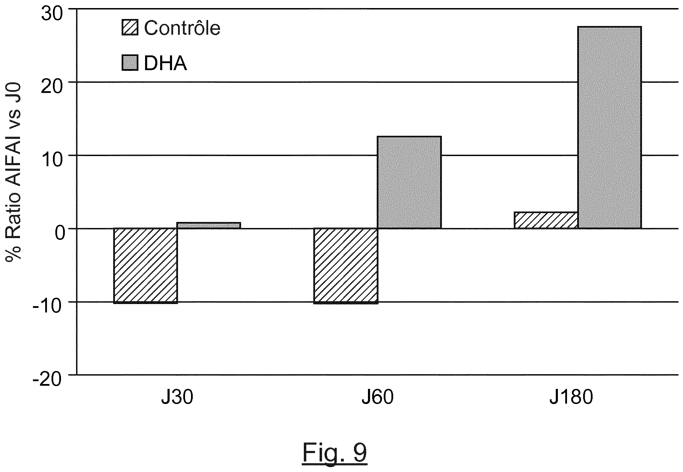

Ces résultats corroborent ceux présentés à la figure 9. L'index d'acides gras anti-inflammatoires (AIFAI ce qui correspond à la traduction anglaise « anti-inflamatory fatty acids index ») représente la quantité totale d'acides gras ayant une activité anti-inflammatoire. D'après le graphique de la figure 9, l'AIFAI des patients du groupe contrôle est négatif et stable pendant au moins les 60 premiers jours de la cure. Il devient légèrement positif au terme des 6 mois de cure. A l'inverse, l'AIFAI des patients ayant reçu une supplémentation en DHA présente un AIFAI nul et légèrement positif dès le premier mois de cure. Il augmente ensuite régulièrement et devient plus de 12 fois supérieur à celui des patients du groupe contrôle.

### 7. Conclusion

Ces résultats démontrent clairement que la prise de compositions alimentaires et des portions alimentaires selon l'invention, contenant de la DHA, permettent de réduire davantage l'inflammation chez les sujets obèses. Plus exactement, la perte de poids seule ne permet pas de résoudre le problème d'inflammation de bas grade observé chez les patients obèses ou en surpoids. Comme le démontrent les résultats présentés aux figures 1 et 2, la DHA ne permet pas de modifier le poids total perdu ni d'accélérer la perte de poids. En revanche, la consommation quotidienne de DHA permet de réduire considérablement l'inflammation de bas grade des patients. Par conséquent, les sujets ayant suivi une cure d'amaigrissement basée sur les compositions selon l'invention seront moins susceptibles de reprendre du poids à la fin de leur cure que les sujets du groupe contrôle. De plus, les risques de développer certaines pathologies comme le diabète de type II et les troubles cardiovasculaires sont davantage réduits.

## Revendications

1. Composition alimentaire de régime prête à l'emploi destinée à être utilisée pour diminuer l'inflammation chronique et modérée chez le patient obèse ou en surpoids, ladite composition comprenant, par rapport au poids total de la composition :
- entre 70% et 80% en poids de protéines ;
- entre 4% et 6% en poids de glucides; et,
- entre 2% et 5% en poids de lipides;
lesdits lipides incluant entre 0,25% et 2% d'acide docosahexaénoïque (DHA) en poids par rapport audit poids de lipides.

2. Composition alimentaire de régime prête à l'emploi pour utilisation selon la revendication 1 obtenue par le mélange d'une portion alimentaire individuelle de régime avec de l'eau ou obtenue sous la forme d'un aliment prêt à consommer.

3. Composition pour utilisation selon la revendication 2, **caractérisée en ce que** la portion alimentaire individuelle de régime comprend :
- 12,5 g à 20 g, de préférence 14 g à 16 g, de protéines et,
- 0,5 g à 5 g, de préférence 1 g à 5 g, de glucides ; et,
- 0,25 à 6 g, de préférence entre 0,5 et 4 g, de lipides; les dits lipides incluant jusqu'à 80 mg de DHA.

4. Composition pour utilisation selon la revendication 3, **caractérisée en ce que** la portion alimentaire individuelle de régime comprend entre 1 mg et 80 mg de DHA, de préférence entre 10 mg et 50 mg de DHA, de manière davantage préférée entre 25 mg et 50 mg de DHA et de manière préférée entre toutes 50 mg de DHA.

5. Composition pour utilisation selon les revendications 2 à 4, **caractérisée en ce que** la portion alimentaire individuelle de régime a un poids compris entre 5g et 300g, de préférence entre 15g et 200g, de manière davantage préférée entre 20g et 125g.

6. Composition pour utilisation selon les revendications 2 à 5, **caractérisée en ce que** la portion alimentaire individuelle de régime a été réalisée à partir d'ingrédients culinaires dont au moins un contient du DHA.

7. Composition pour utilisation selon la revendication 6, **caractérisée en ce que** ledit ingrédient culinaire contenant du DHA est choisi parmi le jaune d'oeuf, l'huile de poisson, les extraits d'algues, les champignons, le krill ou la combinaison d'au moins deux de ces ingrédients.

8. Composition pour utilisation selon l'une des revendications précédentes, **caractérisée en ce que** la lesdites protéines sont des protéines choisies parmi les protéines de lait, de lactosérum , de soja, d'oeuf ou leur combinaison.

9. Composition pour utilisation selon l'une des revendications précédentes, **caractérisée en ce qu'**elle est administrée oralement pendant une période comprise entre 30 jours et 180 jours par le patient, de préférence entre 60 jours et 180 jours, de manière davantage préférée entre 90 jours et 180 jours.

## Patentansprüche

1. Gebrauchsfertige Diätnahrungsmittelzusammensetzung für die Verwendung zur Verringerung der chronischen und mäßigen Entzündung bei einem fettleibigen oder übergewichtigen Patienten, wobei die Zusammensetzung, bezogen auf das Gesamtgewicht der Zusammensetzung, Folgendes umfasst:
- zwischen 70 Gew.-% und 80 Gew.-% Proteine,
- zwischen 4 Gew.-% und 6 Gew.-% Kohlenhydrate und
- zwischen 2 Gew.-% und 5 Gew.-% Lipide,
wobei die Lipide zwischen 0,25 Gew.-% und 2 Gew.-% Docosahexaensäure (DHA), bezogen auf das Gewicht der Lipide, einschließen.

2. Gebrauchsfertige Diätnahrungsmittelzusammensetzung zur Verwendung nach Anspruch 1, die durch Mischen einer einzelnen Diätnahrungsmittelportion mit Wasser erhalten wird oder in Form eines verzehrfertigen Lebensmittels erhalten wird.

3. Zusammensetzung zur Verwendung nach Anspruch 2, **dadurch gekennzeichnet, dass** die einzelne Diätnahrungsmittelportion Folgendes umfasst:
- 12,5 g bis 20 g, vorzugsweise 14 g bis 16 g, Proteine und
- 0,5 g bis 5 g, vorzugsweise 1 g bis 5 g, Kohlenhydrate und
- 0,25 bis 6 g, vorzugsweise 0,5 g bis 4 g, Lipide, wobei die Lipide bis zu 80 mg DHA einschließen.

4. Zusammensetzung zur Verwendung nach Anspruch 3, **dadurch gekennzeichnet, dass** die einzelne Diätnahrungsmittelportion zwischen 1 mg und 80 mg DHA, vorzugsweise zwischen 10 mg und 50 mg DHA, noch stärker bevorzugt zwischen 25 mg und 50 mg DHA und am stärksten bevorzugt 50 mg DHA umfasst.

5. Zusammensetzung zur Verwendung nach den Ansprüchen 2 bis 4, **dadurch gekennzeichnet, dass** die einzelne Diätnahrungsmittelportion ein Gewicht zwischen 5 g und 300 g, vorzugsweise zwischen 15 g und 200 g, stärker bevorzugt zwischen 20 g und 125 g hat.

6. Zusammensetzung zur Verwendung nach den Ansprüchen 2 bis 5, **dadurch gekennzeichnet, dass** die einzelne Diätnahrungsmittelportion aus Nahrungsmittelzutaten hergestellt ist, von denen mindestens eine DHA enthält.

7. Zusammensetzung zur Verwendung nach Anspruch 6, **dadurch gekennzeichnet, dass** die Nahrungsmittelzutat, die DHA enthält, aus Eigelb, Fischöl, Algenextrakten, Pilzen, Krill oder einer Kombination von mindestens zwei dieser Zutaten ausgewählt ist.

8. Zusammensetzung zur Verwendung nach einem der vorhergehenden Ansprüche, **dadurch gekennzeichnet, dass** es sich bei den Proteinen um Proteine handelt, die aus Milch-, Molke-, Soja-, Eiproteinen oder ihrer Kombination ausgewählt sind.

9. Zusammensetzung zur Verwendung nach einem der vorhergehenden Ansprüche, **dadurch gekennzeichnet, dass** sie oral während eines Zeitraums zwischen 30 Tagen und 180 Tagen, vorzugsweise zwischen 60 Tagen und 180 Tagen, stärker bevorzugt zwischen 90 Tagen und 180 Tagen, vom Patienten eingenommen wird.

## Claims

1. Ready-to-use diet food composition for use to reduce chronic and moderate inflammation in an obese or overweight patient, said composition comprising, as a proportion of the total weight of the composition:
- from 70% to 80% by weight of protein;
- from 4% to 6% by weight of carbohydrates; and,
- from 2% to 5% by weight of lipids;
said lipids including 0.25% to 2% by weight of docosahexaenoic acid (DHA) relative to said weight of lipids.

2. Ready-to-use diet food composition for use according to claim 1, obtained my mixing an individual diet food portion with water or obtained as a ready-to-use aliment.

3. Ready-to-use diet food composition for use according to claim 2, **characterized in that** the individual diet food portion comprises:
- 12,5g to 20g, preferably 14g to 16g, of protein and,
- 0.5g to 5g, preferably 1g to 5g of carbohydrates,
- 0.25g to 6g, preferably 0.5g to 4g of lipids; said lipids including up to 80 mg of DHA.

4. Ready-to-use diet food composition for use according to claim 3, **characterized in that** the individual diet food portion comprises 1mg to 80mg of DHA, preferably 10mg to 50mg of DHA, more preferably 25mg to 50mg of DHA, and still more preferably 50 mg of DHA.

5. Ready-to-use diet food composition for use according to claims 2 to 5, **characterized in that** the individual diet food portion has a weight of 5g to 300g, preferably 15g to 200g, and in a more preferred manner 20g to 125g.

6. Ready-to-use diet food composition for use according to claims 2 to 5, **characterized in that** the individual diet food portion is made out of culinary ingredients, at least one of which contains DHA.

7. Ready-to-use diet food composition for use according to claim 6, **characterized in that** said culinary ingredient containing DHA is chosen from amongst egg yolk, fish oil, seaweed extract, mushrooms, krill or a combination of at least two of these ingredients.

8. Ready-to-use diet food composition for use according to any of the preceding claims, **characterized in that** said protein is protein chosen from among milk proteins, whey or milk serum, soya, egg or a combination thereof.

9. Ready-to-use diet food composition for use according to any preceding claims, **characterized in that** it is administered orally for a period of 30 days to 180 days by the patient, preferably a period of 60 days to 180 days, and more preferably a period of 90 days to 180 days.
